(19) Europäisches Patentamt
European Patent Office
Office européen des brevets

(11) Numéro de publication : **0 490 777 B1**

# (12) FASCICULE DE BREVET EUROPEEN

(45) Date de publication du fascicule du brevet : **13.04.94 Bulletin 94/15**

(51) Int. Cl.$^5$ : **C07C 323/52,** C08G 18/77, C08F 128/04, G02B 1/04

(21) Numéro de dépôt : **91403410.3**

(22) Date de dépôt : **16.12.91**

(54) **Nouveaux composés soufrés et nouveaux polymères obtenus à partir de ces composés soufrés.**

(30) Priorité : **14.12.90 FR 9015977**

(43) Date de publication de la demande : **17.06.92 Bulletin 92/25**

(45) Mention de la délivrance du brevet : **13.04.94 Bulletin 94/15**

(84) Etats contractants désignés : **AT BE CH DE DK ES FR GB GR IT LI LU NL SE**

(56) Documents cités : **Aucun document pertinent relevé**

(73) Titulaire : **RHONE-POULENC CHIMIE**
**25, quai Paul Doumer**
**F-92408 Courbevoie Cédex (FR)**

(72) Inventeur : **Lavault, Sylvie**
**17, rue Jassron**
**F-69003 Lyon (FR)**
Inventeur : **Velleret, Gérard**
**65, avenue Philippe Auguste**
**F-75011 Paris (FR)**

(74) Mandataire : **Ricalens, François et al**
**RHONE-POULENC CHIMIE Direction de la Propriété Industrielle 25, Quai Paul Doumer**
**F-92408 Courbevoie Cedex (FR)**

EP 0 490 777 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux composés soufrés qui sont des esters de polyols d'acides mercapto-carboxyliques.

Elle concerne également l'utilisation de ces esters soufrés pour préparer des polymères présentant un indice de réfraction supérieur à 1,50.

De nombreuses voies ont été proposées pour atteindre ce résultat.

L'une des plus prometteuses est l'utilisation de monomère comportant un ou plusieurs atomes de soufre.

La présente invention consiste tout d'abord dans les composés soufrés de formule générale (I) :

$$\left[ HS - R_1 - COO \right]_n A \qquad (I)$$

dans laquelle :

- $R_1$ représente un radical alkylène, linéaire ou ramifié ; ayant 1 à 3 atomes de carbone ;
- A représente un reste hydrocarboné de valence n, choisi parmi les radicaux de formules :

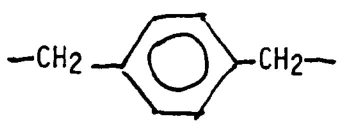

(II)

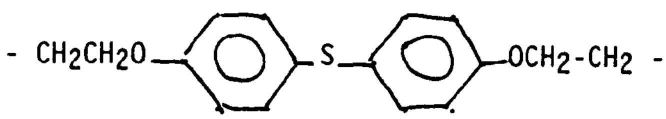

(III)

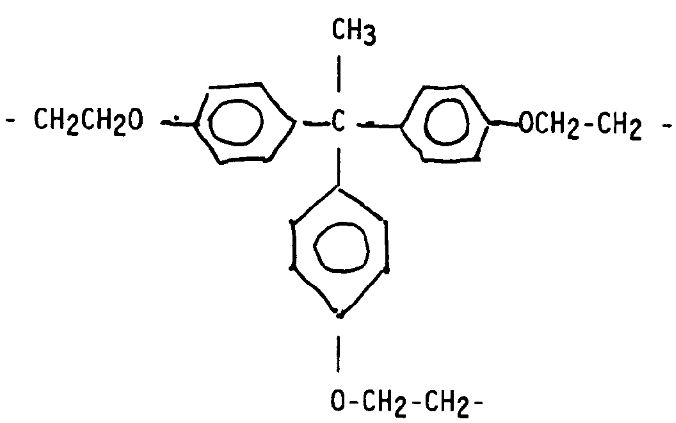

(IV)

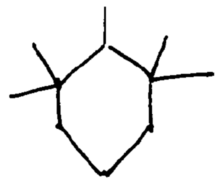

(V)

- n représente 2, 3 ou 5.

Parmi les composés soufrés de formule (I), on préfère le plus souvent ceux pour lesquels $R_1$ représente un radical méthylène, un radical éthylène ou un radical éthylidène.

Ainsi on peut citer le bis(thioglycolate) de benzène-diméthylène-1,4 ; le bis(thioglycolate) de thio-4,4' diphénylène oxyéthyle-1,1' ; le tris(thioglycolate) d'éthane tris(phénylène-4 oxyéthyle-1)-1,1,1 ; le pentakis(thioglycolate) de (tétraméthylène-2,2,6,6 cyclohexyle) ; le bis(mercapto-2 propionate) de benzène-diméthylène-1,4 ; le bis(mercapto-2 propionate) de thio-4,4' diphénylène oxyéthyle-1,1' ; le bis(mercapto-2 propionate) d'éthane tris(phénylène-4 oxyéthyle-1)-1,1,1 ; le pentakis(mercapto-2 propionate) de (tétraméthylène-2,2,6,6 cyclohexyle).

Les composés de formule (I) sont préparés de manière connue par estérification d'un polyol de formule (VI) $A\text{-}(OH)_n$ avec l'acide mercapto-carboxylique de formule (VII) $HS\text{-}R_1COOH$, dans lesquelles les symboles A, n et $R_1$ ont les significations données pour la formule (I), en présence des catalyseurs habituels de ce type de réaction.

Ils peuvent également être préparés par transesterification entre un polyol $A\text{-}(OH)_n$ et un ester d'un alcool de bas point d'ébullition (le plus souvent un ester du méthanol) de l'acide mercaptocarboxylique $HS\text{-}R_1\text{-}COOH$, en présence des catalyseurs habituels de transestérification.

Les composés soufrés de formule (I) conviennent notamment pour la préparation de polythiouréthannes, par réaction entre lesdits composés (I) et au moins un polyisocyanate.

On peut utiliser les composés soufrés de formule (I) en mélanges avec des polythiols connus.

A titre d'exemples de tels polythiols, on peut citer le tétrakis(thioglycolate) de pentaérythrityle, le tétrakis(mercapto-2 propionate) de pentaérythrityle, le tris(thioglycolate) du triméthylolpropane, le tris(mercapto-2 propionate) du triméthylolpropane, le benzène-dithiol-1,2, le benzène-dithiol-1,4, l'hexakis(thioglycolate) du dipentaérythritol, le tris(mercapto-3 propyl)-1,3,5 isocyanurate.

On peut également se référer à d'autres polythiols tels que des composés légers e.g. dimercapto propanol, dithioerithritol, trithioglycérine, tetramercapto-butane, pentaerithrithiol, et à ceux cités dans le brevet EP-A-0 351 073.

On préfère comme constituant thiol autre que ceux selon l'invention, des monomères saturé acyclique non ester porteur d'au moins trois fonctrions réactives à l'égard de isocyanates pour former des liaisons carbamates , parmi lesquels fonctions réactives au moins 40% en nombre sont des groupes mercaptans SH, la proportion desdites fonctions étant d'au moins 45%en masse par rapport a la masse moléculaire du dit monomère.

Il n'y a pas de limitation particulière pour les polyisocyanates servant à préparer lesdits polythiouréthannes.

Ce sont des polyisocyanates proprement dits comme par exemple le toluène-diisocyanate, le diisocyanato-4,4' diphénylméthane, le diisocyanato-4,4' diphénylméthane polymérique, l'hexaméthylène diisocyanate, le méthyl-2 diisocyanato-1,5 pentane, l'éthyl-2 diisocyanato-1,4 butane, l'isophorone-diisocyanate, le xylylène-diisocyanate, le diisocyanato-4,4' dicyclohexylméthane, le xylylène-diisocyanate hydrogéné.

Ce sont également les trimères à cycle isocyanurate ou les biurets de tels polyisocyanates.

Parmi les polyisocyanates, on préférera ceux dans la formule desquels les fonctions -NCO ne sont pas directement liées à un cycle romatique, car les polythiouréthannes préparés à partir des polyisocyanates aromatiques ont tendance à être colorés.

D'autre part, il est bien évident que parmi les polyisocyanates, on choisira ceux qui présentent déjà euxmêmes un indice de réfraction suffisamment élevé, comme par exemple le xylylène-diisocyanate.

Ainsi il est préféré d'utiliser un polyisocyanate ayant un indice de réfraction égal ou supérieur à 1,53.

La réaction entre les composés soufrés de formule (I) et les polyisocyanates se fait dans les conditions habituelles de ce type de réaction.

Généralement on utlise un composé organique de l'étain comme catalyseur, le plus souvent le dilaurate de dibutyl étain.

Les composés soufrés de formule (I) sont utilisés également pour la préparation de polythioéthers par réaction avec au moins un composé présentant au moins deux insaturations éthyléniques.

Comme composés insaturés, on peut citer par exemple le triallylisocyanurate, le triallylcyanurate, le phtalate de diallyle, le divinylbenzène, le styrène, le triméthallylthiocyanurate, le tris-acrylate d'hydroxy-2 éthylcyanurate, le tris-méthacrylate d'hydroxy-2 éthylcyanurate, le tris(allylcarbonate) d'hydroxy-2 éthylcyanurate, le tris(méthallylcarbonate) d'hydroxy-2 éthylcyanurate, le diacrylate du bis(hydroxy-4 phényl)-2,2 propane.

Parmi ces composés insaturés, on préfèrera ceux qui présentent le plus grand nombre d'insaturations éthylèniques et ceux qui possèdent eux-mêmes un indice de réfraction suffisamment élevé.

Si le nombre d'insaturations éthylèniques n'est pas supérieur à 2, on préfèrera mettre en oeuvre un mélange ternaire polythiol/diinsaturé/triacrylate ou triméthacrylate, afin d'avoir un verre dur. Les triacrylates ou triméthacrylates sont par exemple le triacrylate de triméthylolpropane, le triacrylate du pentaérythritol, le triméthacrylate du pentaérythritol, l'hexa-acrylate du dipentaérythritol, l'hexaméthacrylate du dipentaérythritol.

La réaction entre les composés soufrés de formule (I) et les composés à insaturations éthylèniques se fait avec un excès d'insaturations éthylèniques par rapport aux fonctions thiol.

Il a en effet été mis en évidence une certaine lenteur de la polymèrisation, lorsque l'on augmente la quantité de fonctions thiol.

Cette préparation se fait généralement en présence de catalyseurs, tels que les peroxydes et plus généralement les générateurs de radicaux.

Généralement on utilise de 0,1 % à 10 % en poids de catalyseur par rapport au poids du mélange réactionnel.

Les polythiouréthannes et les polythioéthers obtenus à partir des composés soufrés de formule (I) ont des propriétés qui permettent leur utilisation en optique.

En effet ces polymères ont un indice de réfraction supérieur à 1,50 et un nombre d'ABBE supérieur ou égal à 30, ce qui est représentatif d'une faible dispersion.

Ces polymeres constituent seul ou en mélange des matériaux trés utile pour de nombreuses utilisations optiques y compris la fabrication de lentille ophtalmique.

Ces materiaux ne se dégradent pas dans le temps , ni à la lumière, ni aux intemperies(jaunissement ou pertes de propriété mécaniques) et ils presentent une bonne résistance aux chocs à l'abrasion ils sont colorables et facile à démouler.

Parmi les applications optiques, on peut citer la fabrication de disques optiques et de guides d'ondes.

## EXEMPLES

### Exemple 1 : Préparation du bis-thioglycolate de benzène diméthylène-1,4

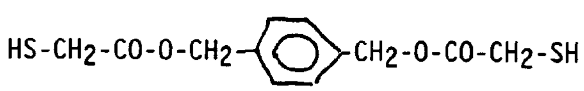

(VIII)

Dans un ballon tricol de 2 litres, muni d'une agitation centrale, d'une gaine thermométrique, d'un "Dean Stark" surmonté d'un réfrigérant sous couverture d'azote, on charge :

- 138,2 g (1,0 mol) de benzène diméthanol-1,4
- 239,5 g (2,6 mol) d'acide thioglycolique distillé
- 3,5 g d'acide paratoluène sulfonique
- 1000 $cm^3$ de toluène.

On chauffe à reflux (110-112°C) sous agitation pendant 1 h 45 : on recueille 37 $cm^3$ d'eau dans la partie inférieure du "Dean Stark".

On laisse refroidir le mélange réactionnel jusqu'à température ambiante, puis on le lave à l'aide de 5 x 500 $cm^3$, puis de 2 x 1000 $cm^3$ d'eau.

La phase organique est séchée sur $Na_2SO_4$, filtrée, puis concentrée sous pression réduite.

On obtient ainsi 274 g (rendement de 96 % environ par rapport au diol de départ) d'un liquide incolore, visqueux, ayant un indice de réfraction $n_D$ = 1,5665.

Le spectre obtenu par RMN du proton correspond à la structure attendue.

Le dosage des fonctions -SH par la soude 0,1 N donne 692 milliéquivalents (meq) pour 100 g.

## FABRICATION DE LENTILLES OPHTALMIQUES

La composition utilisée comprend :

| | | |
|---|---|---|
| Xylylène diisocyanate (XDI) | : | 9,4 g (0,05 mole) |
| Trithioglycérine | : | 2,38 g (0,0172 mole) |
| Bis thioglycolate de benzène diméthylène-1,4: $(HS-(CH_2)_2-CO-O-CH_2-pC_6H_4-CH_2-OCO-CH_2-SH)$ | | 6,86 g (0,024 mole) |

La proportion molaire de trithioglycérine dans le total molaire des monomères est de 42 %.
Le produit obtenu présente les propriétés suivantes :

- indice de réfraction : 1,65
- constringence (nombre d'Abbe) : 30
- température de transition vitreuse : 95° C

## Autres Fabrications de lentilles

La composition utilisée comprend :

| | |
|---|---|
| Xylylène diisocyanate (XDI) | : 9,4 g (0,05 mole) |
| Dimercapto-propanol (DMP) | : 2,23 g (0,018 mole) |
| Para thioglycolate de benzène dimethylène ( $(HS-CH_2-COO-CH_2-S-pC_6H_4-CH_2-OCO-CH_2-SH)$ | : 10,03 g (0,024 mole) |

La proportion molaire de DMP dans le total molaire des monomères est de 41%.
Le produit obtenu présente les propriétés suivantes :

- indice de réfraction : 1,63
- constringence (nombre d'Abbe) : 33
- température de transition vitreuse : 100° C

Exemple 2 : Préparation du bis-thioglycolate de thio-4,4'-diphénylène oxyéthyle-1,1'

A) Ethoxylation du 4,4'-thiodiphénol en bis(hydroxy-2 éthoxy)-1,1' thio-4,4' diphénylène (IX)

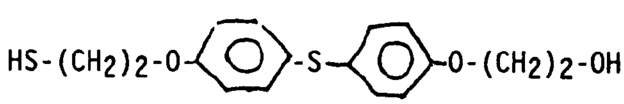
(IX)

Dans un ballon tricol de 2 litres, muni d'une agitation centrale, d'une gaine thermométrique et d'un réfrigérant sous couverture d'azote, on charge :

- 196,4 g (0,90 mol) de 4,4'-thiodiphénol
- 194,3 g (2,21 mol) de carbonate d'éthylène
- 3,9 g de carbonate de potassium
- 900 $cm^3$ de toluène.

On chauffe sous agitation à reflux (112°C environ) pendant 24 heures.

Le mélange réactionnel est alors filtré à chaud ; le filtrat cristallise en refroidissant.

On essore le solide qui a précipité sur un filtre en verre fritté, on le lave à l'éther de pétrole (2 x 1000 $cm^3$), puis à l'eau (2 x 1000 $cm^3$)

Il est ensuite séché sous pression réduite à 60-80°C.

On obtient 257,2 g (rendement de 93 % par rapport au thiodiphénol engagé) d'une poudre blanche dont le point de fusion est de 95-97°C.

La structure attendue est confirmée par RMN du proton.

B) Préparation du bis-thioglycolate de thio-4,4' diphénylène oxyéthyle-1,1'

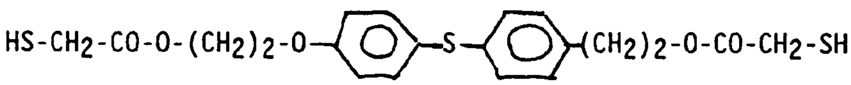

(X)

Dans un ballon tricol de 2 litres, muni d'une agitation centrale, d'une gaine thermométrique, d'un "Dean Stark" surmonté d'un réfrigérant sous couverture d'azote, on charge :

- 245,1 g (0,8 mol) de bis(hydroxy-2 éthoxy)-1,1' thio-4,4' diphénylène préparé en A
- 191,6 g (2,08 mol) d'acide thioglycolique distillé
- 2,0 g d'acide paratoluène sulfonique
- 1000 cm$^3$ de toluène.

On chauffe à reflux (110-112°C) sous agitation pendant 1 h 30 : on recueille 29 cm$^3$ d'eau dans la partie inférieure du "Dean Stark".

On laisse refroidir le mélange réactionnel jusqu'à température ambiante, puis on le lave à l'aide de 7 x 1000 cm$^3$ d'eau.

On ajoute à la phase organique 80 g de $Na_2SO_4$ et 70 g d'alumine activée. On agite pendant 45 min, puis on ajoute 7 g de Clarcel. On agite alors pendant 15 min à 8000 tours/min.

On filtre et on concentre sous pression réduite.

On obtient ainsi 334 g (rendement de 92 % environ par rapport au diol de départ) d'un liquide incolore, visqueux, ayant un indice de réfraction $n_D$ = 1,608, qui cristallise peu à peu en un produit blanc solide.

Le spectre obtenu par RMN du proton correspond à la structure attendue.

Le dosage des fonctions -SH par la soude 0,1 N donne 423 meq SH/100 g.

**FABRICATION DE LENTILLES OPHTALMIQUES**

La composition utilisée comprend :

| | | |
|---|---|---|
| Xylylène diisocyanate (XDI) | : | 9,4 g (0,05 mole) |
| Trithioglycérine | : | 2,38 g (0,0266 mole) |
| para-bis-thioglycolate de thio-4,4' diphénylène oxyéthyle-1,1' | : | 10,03g (0,024 mole) |

La proportion molaire de trithioglycérine dans le total molaire des monomères est de 42 %.

Le produit obtenu présente les propriétés suivantes :

- indice de réfraction : 1,66
- constringence (nombre d'Abbe) : 29
- température de transition vitreuse : 100° C

**Autres Fabrications de lentilles**

La composition utilisée comprend :

| | | |
|---|---|---|
| Xylylène diisocyanate (XDI) | : | 9,4 g (0,05 mole) |
| Dimercapto-propanol (DMP) | : | 2,23 g (0,018 mole) |
| para-bis-thioglycolate de thio-4,4' diphénylène oxyéthyle-1,I' | : | 10,03 g (0,024 mole) |

La proportion molaire de DMP dans le total molaire des monomères est de 43%.
Le produit obtenu présente les propriétés suivantes :

- indice de réfraction : 1,63
- constringence (nombre d'Abbe) : 32
- température de transition vitreuse : 100° C

Exemple 3 : Préparation du A) Ethoxylation du tris(hydroxy-4 phényl)-1,1,1 éthane en tris[(hydroxy-2 éthoxy)-1 phényl]-1,1,1 éthane (XI)

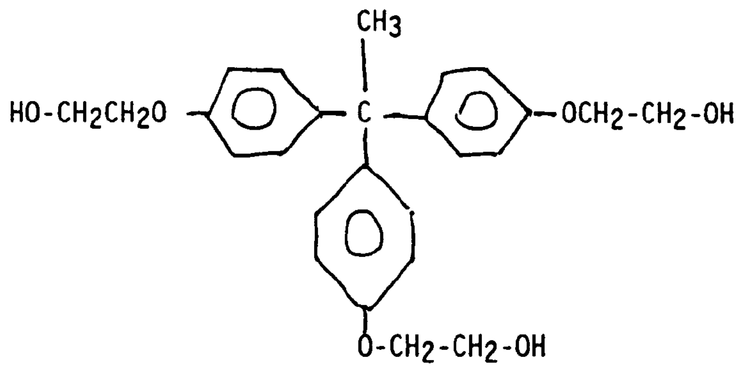

(XI)

Dans un ballon tricol de 0,5 litre, muni d'une agitation centrale, d'une gaine thermométrique et d'un réfrigérant sous couverture d'azote, on charge :

- 49,2 g (0,16 mol) de tris(hydroxy-4 phényl)-1,1,1 éthane
- 50,8 g (0,576 mol) de carbonate d'éthylène
- 1 g de carbonate de potassium
- 250 cm³ de toluène
- 300 cm³ de dioxanne.

On chauffe sous agitation à reflux pendant 36 heures.

Le mélange réactionnel est alors filtré à chaud ; le filtrat (rose-violet) est concentré sous pression réduite. Le résidu coloré commence à cristalliser.

On le reprend par 250 cm³ de dichlorométhane.

On l'essore sur un filtre en verre fritté, on le lave au dichlorométhane (400 cm³).

Il est ensuite séché sous pression réduite.

On obtient 63,4 g (rendement de 90 % par rapport au triphénol engagé) d'une poudre ocre-clair dont le point de fusion est de 50°C.

La structure attendue est confirmée par RMN du proton.

B) Préparation du tris-thioglycolate d'éthane tris (phénylène-4 oxyéthyle-1)-1,1,1

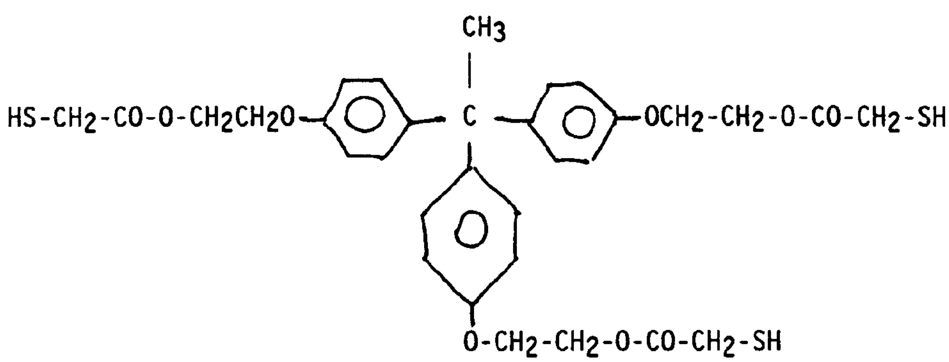

(XII)

Dans un ballon tricol de 250 cm³, muni d'une agitation centrale, d'une gaine thermométrique, d'un "Dean Stark" surmonté d'un réfrigérant sous couverture d'azote, on charge :

- 43,9 g (0,1 mol) de tris[(hydroxy-2 éthoxy)-1 phényl]-1,1,1 éthane préparé en 3 A
- 48 g (0,52 mol) d'acide thioglycolique distillé
- 0,2 g d'acide paratoluène sulfonique
- 100 $cm^3$ de toluène.

On chauffe à reflux sous agitation pendant 1 h 30.

On laisse refroidir le mélange réactionnel jusqu'à température ambiante, puis on le lave à l'aide de 5 x 100 $cm^3$ d'eau.

La phase organique est séchée sur $Na_2SO_4$ et traitée au carbone actif (5 g) à chaud, puis est concentrée sous pression réduite.

On obtient ainsi 59,7 g (rendement de 87 % environ par rapport au triol de départ) d'un liquide trouble, très légèrement jaune et très visqueux, ayant un indice de réfraction $n_D$ = 1,5985.

Le spectre obtenu par RMN du proton correspond à la structure attendue.

Le dosage des fonctions -SH par la soude 0,1 N donne 432 meq SH/100 g.

**FABRICATION DE LENTILLES OPHTALMIQUES**

La composition utilisée comprend :

Xylylène diisocyanate (XDI) : 9,4 g (0,05 mole)

Trithioglycérine : 0,84 g (0,0266 mole)

tris-thioglycolate d'éthane tris (phénylène-4 oxyéthyle-1)-1,1,1 : 14,5 g (0,022 mole)

( $(HS-(CH_2)_2-O-CO-CH_2-S)_2$ $HC-pC_6H_4-CH$ $(-S-CH_2-COO-(CH_2)_2-SH)_2$ )

La proportion molaire de trithioglycérine dans le total molaire des monomères est de 21 %.

Le produit obtenu présente les propriétés suivantes :

- indice de réfraction : 1,66
- constringence (nombre d'Abbe) : 30
- température de transition vitreuse : 100° C

**Autres Fabrications de lentilles**

La composition utilisée comprend :

Xylylène diisocyanate (XDI) : 9,4 g (0,05 mole)

Dimercapto-propanol (DMP) : 0,98 g (0,007 mole)

tris-thioglycolate d'éthane tris (phénylène-4 oxyéthyle-1)-1,1,1 : 15,84 g (0,024 mole)

La proportion molaire de DMP dans le total molaire des monomères est de 27%.

Le produit obtenu présente les propriétés suivantes :

- indice de réfraction : 1,64
- constringence (nombre d'Abbe) : 30
- température de transition vitreuse : 115° C

Exemple 4 : Préparation du pentakis polyglycolate de (tétraméthylène-2,2,6,6 cyclohexyle )

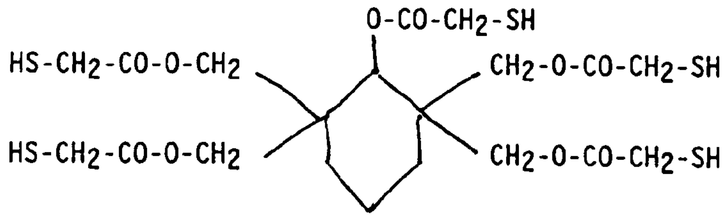

(XIII)

Dans un ballon tricol de 100 $cm^3$, muni d'une agitation centrale, d'une gaine thermométrique, d'un "Dean Stark" surmonté d'un réfrigérant sous couverture d'azote, on charge :

- 11 g (0,05 mol) de tétra(hydroxyméthyl)-2,2,6,6 cyclohexanol
- 34,5 g (0,375 mol) d'acide thioglycolique distillé
- 0,15 g d'acide paratoluène sulfonique
- 50 $cm^3$ de toluène.

On chauffe à reflux (110-115°C) sous agitation pendant 6 h : on recueille 4,6 $cm^3$ d'eau dans la partie inférieure du "Dean Stark".

On laisse refroidir le mélange réactionnel jusqu'à température ambiante, puis on le lave à l'aide de 7 x 30 $cm^3$ d'eau.

La phase organique est séchée sur $Na_2SO_4$, filtrée, puis concentrée sous pression réduite.

On obtient ainsi 24,4 g (rendement de 81 % environ par rapport au polyol de départ) d'un liquide incolore, visqueux, ayant un indice de réfraction $n_D$ = 1,558.

Le dosage des fonctions -SH par la soude 0,1 N donne 767 meq SH/100 g (théorie 968 meq SH/100 g).

Par analyse en spectrométrie de masse, on détermine la présence de 2 produits :

- le produit de formule (XIII) attendu (majoritaire)
- le produit de formule (XIV) suivante :

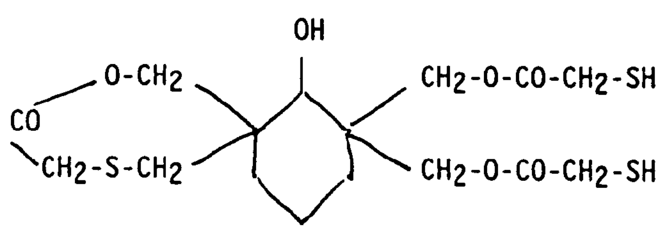

(XIV)

On purifie 10 g de ce mélange de produits par chromatographie liquide haute performance préparative.

On récupère les 2 produits purs (XIII) et (XIV) :

- 5,6 g du produit (XIII) : $n_D$ = 1,545
- 3,5 g du produit (XIV) : $n_D$ = 1,5455

Ces produits sont caractérisés en spectométrie de masse.

**FABRICATION DE LENTILLES OPHTALMIQUES**

La composition utilisée comprend :

Xylylène diisocyanate (XDI) : 9,4 g (0,05 mole)

trithioglycérine : 0,84 g (0,0266 mole)

pentakis polyglycolate de (tétraméthylène-2,2,6,6 cyclohexyle )
5,9 g (0,01 mole)

La proportion molaire de trithioglycérine dans le total molaire des monomères est de 63 %.

Le produit obtenu présente les propriétés suivantes :

- indice de réfraction : 1,65
- constringence (nombre d'Abbe) : 32
- température de transition vitreuse : 120° C

**Autres Fabrications de lentilles**

La composition utilisée comprend :

Xylylène diisocyanate (XDI) : 9,4 g (0,05 mole)
Dimercapto-propanol (DMP) : 0,99 g (0,008 mole)
pentakis polyglycolate de (tétraméthylène-2,2,6,6 cyclohexyle ) : 8,85 g (0,015mole)

La proportion molaire de DMP dans le total molaire des monomères est de 35 %.

Le produit obtenu présente les propriétés suivantes :

- indice de réfraction : 1,62
- constringence (nombre d'Abbe) : 34
- température de transition vitreuse : 135° C

<u>Exemple 5 : Obtention d'un verre à partir du composé de formule (VIII) (préparé dans l'exemple 1)</u>

On mélange à température ambiante :

- 50 g de triallylisocyanurate
- 50 g de composé soufré de formule (VIII)
- 3 g de peroxycarbonate de cyclohexyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 16 h : on élève la température à 35°C en 10 min, puis à 55°C en 1 h, puis à 60°C en 1 h ; on maintient pendant 10 h à 60°C, puis on élève la température à 65°C en 20 min, puis à 70°C en 40 min, à 80°C en 30 min, à 90°C en 1 h et enfin à 100°C en 1 h. On laisse ensuite la température revenir à 20°C.

Le verre obtenu est transparent.

Ses caractéristiques sont les suivantes :

- densité = 1,286
- indice de réfraction =1,568
- nombre d'ABBE = 44

(Un verre à base d'allylcarbonate de diéthylèneglycol présente une densité de 1,32, un indice de réfraction de 1,50 et un nombre d'ABBE de 60).

<u>Exemple 6 : Obtention d'un verre à partir du composé de formule (VIII) (préparé dans l'exemple 1)</u>

On mélange à température ambiante :

- 25 g de triallylisocyanurate
- 25 g de triacrylate de triméthylolpropane
- 50 g de composé soufré de formule (VIII)
- 3 g de peroxycarbonate de cyclohexyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique de 16 h : on élève la température à 35°C en 10 min, puis à 55°C en 1 h 50, puis à 65°C en 12 h, puis on élève la température à 70°C en 1 h et enfin à 80°C en 1 h. On laisse ensuite la température revenir à 20°C.

Le verre obtenu est transparent.

Ses caractéristiques sont les suivantes :
- densité = 1,27
- indice de réfraction = 1,565
- nombre d'ABBE = 40

Exemple 7 : Obtention d'un verre à partir du composé de formule (X) (préparé dans l'exemple 2B)

On mélange à température ambiante :
- 50 g de triallylisocyanurate
- 50 g de composé soufré de formule (X)
- 3 g de peroxycarbonate de cyclohexyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 16 h : on élève la température à 35°C en 10 min, puis à 55°C en 1 h, puis à 60°C en 1 h ; on maintient pendant 10 h à 60°C, puis on élève la température à 65°C en 20 min, puis à 70°C en 40 min, à 80°C en 30 min, à 90°C en 1 h et enfin à 100°C en 1 h. On laisse ensuite la température revenir à 20°C.

Le verre obtenu est transparent.

Ses caractéristiques sont les suivantes :
- densité = 1,271
- indice de réfraction = 1,571
- nombre d'ABBE = 35

Exemple 8 : Obtention d'un verre à partir du composé de formule (X) (préparé dans l'exemple 2B)

On mélange à température ambiante :
- 25 g de triallylisocyanurate
- 25 g de triacrylate de triméthylolpropane
- 50 g de composé soufré de formule (X)
- 3 g de peroxycarbonate de cyclohexyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique de 16 h : on élève la température à 35°C en 10 min, puis à 55°C en 1 h 50, puis à 65°C en 12 h, puis on élève la température à 70°C en 1 h et enfin à 80°C en 1 h. On laisse ensuite la température revenir à 20°C.

Le verre obtenu est transparent.

Ses caractéristiques sont les suivantes :
- densité = 1,27
- indice de réfraction = 1,57
- nombre d'ABBE = 35

Exemple 9 : Obtention d'un verre à partir du composé de formule (XII) (préparé dans l'exemple 3B)

On mélange à température ambiante :
- 50 g de triallylisocyanurate
- 50 g de composé soufré de formule (XII)
- 3 g de triméthyl-3,3,5 perhexanoate de tertiobutyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 19 h : on élève la température à 40°C en 8 min, puis à 60°C en 10 min, puis à 85°C en 1 h ; puis on élève la température de 85 à 95°C pendant 16 h et enfin à 105°C en 1 h ; on maintient encore pendant 1 h à 105°C. On laisse ensuite la température revenir à 20°C.

Le verre obtenu est transparent.

Ses caractéristiques sont les suivantes :
- densité = 1,30
- indice de réfraction = 1,582
- nombre d'ABBE = 30

Exemple 10 : Obtention d'un verre à partir du composé de formule (XIII) (préparé dans l'exemple 4)

On mélange à température ambiante :
- 50 g de triallylisocyanurate
- 50 g de composé soufré de formule (XIII)
- 3 g de triméthyl-3,3,5 perhexanoate de tertiobutyle

Le mélange homogène obtenu est introduit dans un espace délimité par un joint circulaire en "VITON" (diamètre 20 mm, épaisseur 2 mm) placé entre deux plaques de verre minéral. La réaction de polymérisation a lieu en étuve programmée, selon un cycle thermique d'environ 19 h : on élève la température à 40°C en 8 min, puis à 60°C en 10 min, puis à 85°C en 1 h ; puis on élève la température de 85 à 95°C pendant 16 h et enfin à 105°C en 1 h ; on maintient encore pendant 1 h à 105°C. On laisse ensuite la température revenir à 20°C.

Le verre obtenu est transparent.

Ses caractéristiques sont les suivantes :
- densité = 1,30
- indice de réfraction = 1,55
- nombre d'ABBE = 40

Exemple 11 : Obtention d'un verre à partir du composé de formule (XII) (préparé dans l'exemple 3B)

On mélange à température ambiante :
- 20 g de triallylisocyanurate
- 40 g de divinylbenzène
- 40 g de composé soufré de formule (XII)
- 3 g de triméthyl-3,3,5 perhexanoate de tertiobutyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 19 h : on élève la température à 40°C en 8 min, puis à 60°C en 10 min, puis à 85°C en 1 h ; puis on élève la température de 85 à 95°C pendant 16 h et enfin à 105°C en 1 h ; on maintient encore pendant 1 h à 105°C. On laisse ensuite la température revenir à 20°C.

Le verre obtenu est transparent.

Ses caractéristiques sont les suivantes :
- densité = 1,19
- indice de réfraction = 1,589
- nombre d'ABBE = 30

Exemple 12 : Obtention d'un verre à partir du composé de formule (XII) (préparé dans l'exemple 3B)

On mélange à température ambiante :
- 20 g de triallylisocyanurate
- 40 g de diacrylate de bisphénol A (bisphénol A = bis[hydroxy-4 phényl]-2,2 propane)
- 40 g de composé soufré de formule (XII)
- 3 g de triméthyl-3,3,5 perhexanoate de tertiobutyle

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon un cycle thermique d'environ 19 h : on élève la température à 40°C en 8 min, puis à 60°C en 10 min, puis à 85°C en 1 h ; puis on élève la température de 85 à 95°C pendant 16 h et enfin à 105°C en 1 h ; on maintient encore pendant 1 h à 105°C. On laisse ensuite la température revenir à 20°C.

Le verre obtenu est transparent.

Ses caractéristiques sont les suivantes :
- densité = 1,29
- indice de réfraction = 1,583
- nombre d'ABBE = 30

Exemple 13 : Obtention d'un verre à partir du composé de formule (XII) (préparé dans l'exemple 3B)

On mélange à température ambiante :

- 1,22 g de xylylène diisocyanate
- 3,0 g de composé soufré de formule (XII)
- 0,001 g de dilaurate de dibutylétain.

Le mélange homogène obtenu est introduit dans un espace délimité par un joint circulaire en "VITON" (diamètre 20 mm, épaisseur 2 mm) placé entre deux plaques de verre minéral.

La réaction de polymérisation a lieu en étuve programmée, selon le cycle thermique suivant :

- 3 heures de chauffage pour passer de la température ambiante à 80°C,
- 3 heures de maintien à 80°C,
- 2 heures de chauffage pour passer de 80°C à 130°C,
- 3 heures de maintien à 130°C.,

On laisse ensuite la température redescendre à 20°C.
Le verre obtenu est jaune et transparent.

Ses caractéristiques sont les suivantes :

- indice de réfraction = 1,6118
- nombre d'ABBE = 29

## Revendications

1. Composés soufrés de formule générale (I) :

$$[HS - R_1 - COO]_n A \qquad (I)$$

dans laquelle :

- $R_1$ représente un radical alkylène, linéaire ou ramifié ; ayant 1 à 3 atomes de carbone ;
- A représente un reste hydrocarboné de valence n, choisi parmi les radicaux de formules :

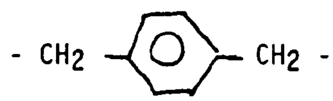

(II)

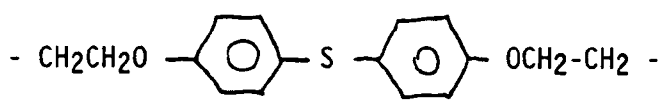

(III)

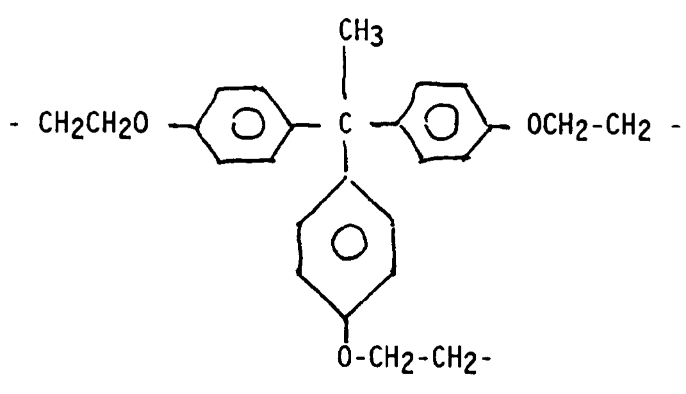

(IV)

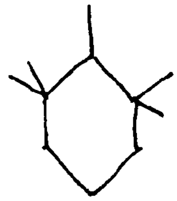

(V)

- n représente 2, 3 ou 5.

2. Composés de formule (I) selon la revendication 1, caractérisés en ce que $R_1$ représente un radical méthylène, un radical éthylène ou un radical éthylidène.

3. Composés de formule (I) selon l'une des revendications 1 ou 2, caractérisés en ce qu'ils sont choisis parmi le bis(thioglycolate) de benzène-diméthylène-1,4 ; le bis(thioglycolate) de thio-4,4' diphénylène oxyéthyle-1,1' ; le tris(thioglycolate) d'éthane tris(phénylène-4 oxyéthyle-1)-1,1,1 ; le pentakis (thioglycolate) de (tétraméthylène-2,2,6,6 cyclohexyle) ; le bis(mercapto-2 propionate) de benzène-diméthylène-1,4 ; le bis(mercapto-2 propionate) de thio-4,4' diphénylène oxyéthyle-1,1' ; le bis(mercapto-2 propionate) d'éthane tris(phénylène-4 oxyéthyle-1)-1,1,1 ; le pentakis(mercapto-2 propionate) de (tétraméthylène-2,2,6,6 cyclohexyle).

4. Polythiouréthannes caractérisés en ce qu'ils sont préparés par réaction entre les composés soufrés de formule (I) selon l'une des revendications 1 à 3 et au moins un polyisocyanate avantageusement choisi parmi ceux dans la formule desquels les fonctions -NCO ne sont pas directement liées à un cycle aromatique.

5. Polythiouréthannes selon la revendication 4, caractérisés en ce que l'on utilise au moins un polyisocyanate tel que le toluène-diisocyanate, le diisocyanato-4,4' diphénylméthane, le diisocyanato-4,4' diphénylméthane polymérique, l'hexaméthylène diisocyanate, le méthyl-2 diisocyanato-1,5 pentane, l'éthyl-2 diisocyanato-1,4 butane, l'isophorone-diisocyanate, le xylilène-diisocyanate, le diisocyanato-4,4' dicyclohexylméthane, le xylilène-diisocyanate hydrogéné, les trimères à cycle isocyanurate et les biurets de tels polyisocyanates.

6. Polythiouréthannes selon l'une des revendications 4 ou 5, caractérisés en ce que ils comportent, outre au moins un composés soufrés de formule (I) selon l'une des revendications 1 à 3, au mois un monomère avantageusement porteur d'au moins trois fonctions réactives avec les isocyanates pour donner des fonctions carbamates, lesdites fonctions réactives etant de preference telle que quelle soient constituées d'au moins 40% de fonction mercaptan (-SH) et avantageusement la masse des dites fonctions etant d'au moins 45% par rapport à la masse des monomères.

7. Polythiouréthannes selon l'une des revendications 4 à 6, caractérisés en ce que le polyisocyanate est choisi parmi ceux qui ont un indice de réfraction égal ou supérieur à 1,53.

8. Polythioéthers caractérisés en ce qu'ils sont préparés par réaction entre les composés soufrés de formule (I) selon l'une des revendications 1 à 3 et au moins un composé présentant au moins deux insaturations éthyléniques.

9. Polythioéthers selon la revendication 8, caractérisés en ce que le composé à insaturation éthylènique est choisi parmi le triallylisocyanurate, le triallylcyanurate, le phtalate de diallyle, le divinylbenzène, le styrène, le triméthallylthiocyanurate, le tris-acrylate d'hydroxy-2 éthylcyanurate, le tris-méthacrylate d'hydroxy-2 éthylcyanurate, le tris(allylcarbonate) d'hydroxy-2 éthylcyanurate, le tris(méthallylcarbonate) d'hydroxy-2 éthylcyanurate, le diacrylate du bis(hydroxy-4 phényl)-2,2 propane.

10. Polythioéthers selon l'une des revendications 8 ou 9, caractérisés en ce que l'on met en oeuvre un mélange ternaire polythiol/diinsaturé/triacrylate ou triméthacrylate tel que le triacrylate de triméthylolpropane, le triacrylate du pentaérythritol, le triméthacrylate du pentaérythritol, l'hexa-acrylate du dipentaérythritol, le hexaméthacrylate du dipentaérythritol.

**11.** Polythioéthers selon l'une des revendications 8 à 10, caractérisés en ce que la réaction entre les composés soufrés de formule (I) et les composés à insaturations éthylèniques se fait avec un excès d'insaturations éthylèniques par rapport aux fonctions thiol.

**12.** Polythioéthers selon l'une des revendications 8 à 11, caractérisés en ce que leur préparation se fait en présence de catalyseurs, tels que les peroxydes et plus généralement les générateurs de radicaux.

**13.** Polythioéthers selon l'une des revendications 8 à 12, caractérisés en ce que lors de leur préparation on utilise de 0,1 % à 10 % en poids de catalyseur par rapport au poids du mélange réactionnel.

**14.** Matériau utile pour application optique comportant au moins pour moitié, de preférence pour 3/4, avantageusement pour 9/10 des polymères formés à partir des composés de la revendication 1, les polymères étant avantageusement choisi parmi ceux visé aux revendications 4 à 13.

**Patentansprüche**

**1.** Schwefelverbindungen der allgemeinen Formel (I):

$$\left[ HS - R_1 - COO \right]_n A$$

in der:

- $R_1$ einen linearen oder verzweigten Alkylenrest mit 1 bis 3 Kohlenstoffatomen darstellt;
- A einen Kohlenwasserstoffrest der Wertigkeit n darstellt, der ausgewählt ist aus den Resten der Formeln:

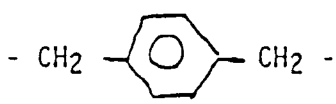

(II)

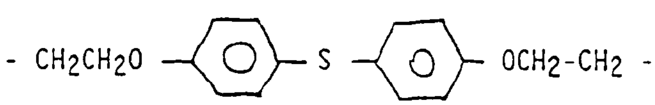

(III)

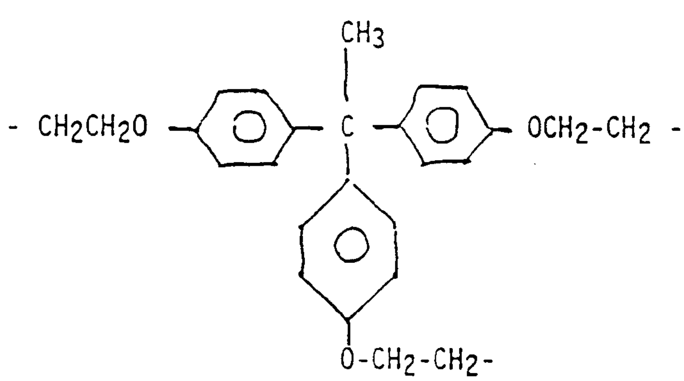

(IV)

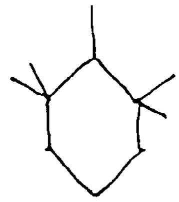

(V)

- n 2, 3 oder 5 darstellt.

2. Verbindungen der Formel (I) nach Anspruch 1, dadurch gekennzeichnet, daß $R_1$ einen Methylenrest, einen Ethylenrest oder einen Ethylidenrest darstellt.

3. Verbindungen der Formel (I) nach einem der Ansprüche 1 oder 2, dadurch gekennzeichnet, daß sie ausgewählt sind aus Benzol-1,4-dimethylen-bis(thioglycolat); 4,4'-Thiodiphenylen-1,1'-oxyethyl-bis(thioglycolat); Ethan-1,1,1-tris(4-phenylen-1-oxyethyl)tris(thioglycolat); (2,2,6,6-Tetramethylencyclohexyl)pentakis-(thioglycolat); Benzol-1,4-dimethylen-bis(2-mercaptopropionat); 4,4'-Thiodiphenylen-1,1'-oxyethyl-bis(2-mercaptopropionat); Ethan-1,1,1-tris(4-phenylen-1-oxyethyl)bis(2-mercaptopropionat); (2,2,6,6-Tetramethylencyclohexyl)pentakis(2-mercaptopropionat).

4. Polythiourethane, dadurch gekennzeichnet, daß sie durch Reaktion zwischen den Schwefelverbindungen der Formel (I) nach einem der Ansprüche 1 bis 3 und mindestens einem Polyisocyanat, das vorteilhafterweise aus denjenigen ausgewählt ist, in deren Formel die Funktionen -NCO nicht direkt an einen aromatischen Ring gebunden sind, hergestellt sind.

5. Polythiourethane nach Anspruch 4, dadurch gekennzeichnet, daß man mindestens ein Polyisocyanat derart wie Toluoldiisocyanat, 4,4'-Diisocyanatodiphenylmethan, polymeres 4,4'-Diisocyanatodiphenylmethan, Hexamethylendiisocyanat, 2-Methyl-1,5-diisocyanatopentan, 2-Ethyl-1,4-diisocyanatobutan, Isophorondiisocyanat, Xylylendiisocyanat, 4,4'-Diisocyanatodicyclohexylmethan, hydriertes Xylylendiisocyanat, die Trimeren mit Isocyanuratring und die Biurete derartiger Polyisocyanate verwendet.

6. Polythiourethane nach einem der Ansprüche 4 oder 5, dadurch gekennzeichnet, daß sie über mindestens eine Schwefelverbindung der Formel (I) nach einem der Ansprüche 1 bis 3 hinaus mindestens ein Monomer umfassen, das vorteilhafterweise Träger von mindestens drei mit Isocyanaten reaktiven Funktionen ist, um Carbamat-Funktionen zu ergeben, wobei die reaktiven Funktionen vorzugsweise derart sind, daß sie aus mindestens 40% Mercaptan-Funktion (-SH) bestehen und vorteilhafterweise die Masse besagter Funktionen mindestens 45%, bezogen auf die Masse der Monomeren, beträgt.

7. Polythiourethane nach einem der Ansprüche 4 bis 6, dadurch gekennzeichnet, daß das Polyisocyanat ausgewählt ist aus denjenigen, die einen Brechungsindex gleich oder oberhalb von 1,53 aufweisen.

8. Polythioether, dadurch gekennzeichnet, daß sie durch Reaktion zwischen den Schwefelverbindungen der Formel (I) nach einem der Ansprüche 1 bis 3 und mindestens einer Verbindung, die mindestens zwei ethylenische Unsättigungen aufweist, hergestellt sind.

9. Polythioether nach Anspruch 8, dadurch gekennzeichnet, daß die Verbindung mit ethylenischer Unsättigung ausgewählt ist aus Triallylisocyanurat, Triallylcyanurat, Diallylphthalat, Divinylbenzol, Styrol, Trimethallylthiocyanurat, dem Trisacrylat von 2-Hydroxyethylcyanurat, dem Trismethacrylat von 2-Hydroxyethylcyanurat, dem Tris(allylcarbonat) von 2-Hydroxyethylcyanurat, dem Tris(methallylcarbonat) von 2-Hydroxyethylcyanurat, dem Diacrylat von 2,2-Bis(4-hydroxyphenyl)propan.

10. Polythioether nach einem der Ansprüche 8 oder 9, dadurch gekennzeichnet, daß man eine ternäre Mischung Polythiol/zweifach ungesättigte Verbindung/Triacrylat oder Trimethacrylat, wie Triacrylat von Trimethylolpropan, Triacrylat von Pentaerythrit, Trimethacrylat von Pentaerythrit, Hexaacrylat von Dipentaerythrit, Hexamethacrylat von Dipentaerythrit, verwendet.

11. Polythioether nach einem der Ansprüche 8 bis 10, dadurch gekennzeichnet, daß die Reaktion zwischen den Schwefelverbindungen der Formel (I) und den Verbindungen mit ethylenischen Unsättigungen mit einem Überschuß an ethylenischen Unsättigungen in bezug auf die Thiol-Funktionen durchgeführt wird.

12. Polythioether nach einem der Ansprüche 8 bis 11, dadurch gekennzeichnet, daß ihre Herstellung in Gegenwart von Katalysatoren, wie Peroxiden und allgemein Radikalerzeugern, durchgeführt wird.

13. Polythioether nach einem der Ansprüche 8 bis 12, dadurch gekennzeichnet, daß man während ihrer Herstellung 0,1 bis 10 Gew.-% Katalysator, bezogen auf das Gewicht der Reaktionsmischung, verwendet.

14. Material, das zur optischen Anwendung nützlich ist, welches mindestens zur Hälfte, bevorzugt zu 3/4, vorteilhafterweise zu 9/10, der Polymeren, die ausgehend -von den Verbindungen des Anspruchs 1 gebildet sind, die Polymere, die vorteilhafterweise aus den in den Ansprüchen 4 bis 13 betroffenen ausgewählt sind, umfaßt.

## Claims

1. Sulphur compounds of general formula (I):

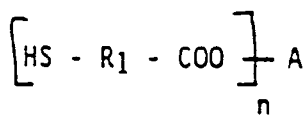

(I)

in which:

- $R_1$ denotes a linear or branched alkylene radical containing 1 to 3 carbon atoms,
- A denotes a hydrocarbon residue of valency n, chosen from the radicals of formulae:

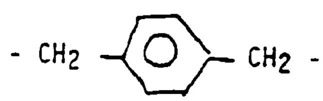

(II)

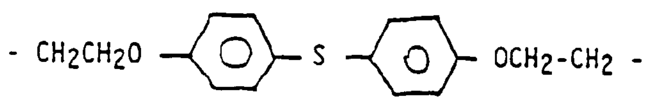

(III)

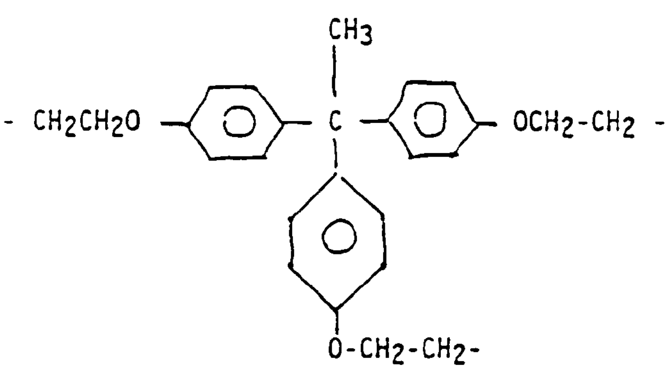

(IV)

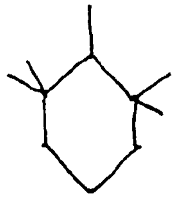

(V)

- n denotes 2, 3 or 5.

2. Compounds of formula (I) according to Claim 1, characterized in that $R_1$ denotes a methylene radical, an ethylene radical or an ethylidene radical.

3. Compounds of formula (I) according to either of Claims 1 and 2, characterized in that they are chosen from 1,4-benzenedimethylene di(thioglycolate), 4,4'-thiodiphenylene-1,1'-oxyethyl di(thioglycolate), 1,1,1-tris(4-phenylene-1-oxyethyl)ethane tri(thioglycolate), 2,2,6,6-tetramethylenecyclohexyl penta(thioglycolate), 1,4-benzenedimethylene di(2-mercaptopropionate), 4,4'-thiophenylene-1,1'-oxyethyl di(2-mercaptopropionate), 1,1,1-tris(4-phenylene-1-oxyethyl) di(2-mercapto- propionate) and 2,2,6,6-tetramethylenecyclohexyl penta(2-mercaptopropionate).

4. Polythiourethanes characterized in that they are prepared by a reaction between the sulphur compounds of the formula (I) according to one of Claims 1 to 3 and at least one polyisocyanate advantageously chosen from those in whose formula the -NCO functional groups are not bonded directly to an aromatic ring.

5. Polythiourethanes according to Claim 4, characterized in that at least one polyisocyanate is employed, such as toluene diisocyanate, 4,4'-diisocyanatodiphenylmethane, polymeric 4,4'-diisocyanatodiphenylmethane, hexamethylene diisocyanate, 2-methyl-1,5-diisocyanato- pentane, 2-ethyl-1,4-diisocyanatobutane, isophorone diisocyanate, xylylene diisocyanate, 4,4'-diisocyanatodicyclohexylmethane and hydrogenated xylylene diisocyanate, trimers containing an isocyanurate ring and the biurets of such polyisocyanates.

6. Polythiourethanes according to either of Claims 4 and 5, characterized in that, besides at least one sulphur compound of formula (I) according to one of Claims 1 to 3, they comprise at least one monomer advantageously bearing at least three functional groups that can react with isocyanates to give carbamate functional groups, the said reactive functional groups being preferably such that they consist of at least 40 % of mercaptan (-SH) functional groups and the mass of the said functional groups being advantageously at least 45 % in relation to the mass of the monomers.

7. Polythiourethanes according to one of Claims 4 to 6, characterized in that the polyisocyanate is chosen from those which have a refractive index equal to or higher than 1.53.

8. Polythioethers characterized in that they are prepared by reaction between the sulphur compounds of formula (I) according to one of Claims 1 to 3 and at least one compound containing at least two ethylenic unsaturations.

9. Polythioethers according to Claim 8, characterized in that the compound containing ethylenic unsaturation is chosen from triallyl isocyanurate, triallyl cyanurate, diallyl phthalate, divinylbenzene, styrene, trimethallyl thiocyanurate, 2-hydroxyethylcyanurate triacrylate, 2-hydroxyethylcyanurate trimethacrylate, 2-hydroxyethylcyanurate tris(allylcarbonate), 2-hydroxyethylcyanurate tris(methallylcarbonate) and 2,2-bis(4- hydroxyphenyl)propane diacrylate.

10. Polythioethers according to either of Claims 8 and 9, characterized in that a ternary mixture of polythiol/diunsaturated/triacrylate or trimethacrylate such as trimethylolpropane triacrylate, pentaerythritol triacrylate, pentaerythritol trimethacrylate, dipentaerythritol hexaacrylate or dipentaerythritol hexamethacrylate is used.

11. Polythioethers according to one of Claims 8 to 10, characterized in that the reaction between the sulphur compounds of formula (I) and the compounds containing ethylenic unsaturations takes place with an excess of ethylenic unsaturations relative to the thiol functional groups.

12. Polythioethers according to one of Claims 8 to 11, characterized in that their preparation takes place in the presence of catalysts such as peroxides and, more generally, radical-generators.

13. Polythioethers according to one of Claims 8 to 12, characterized in that during their preparation from 0.1 % to 10 % by weight of catalyst is employed relative to the weight of the reaction mixture.

14. Material usable for optical application, of which at least half, preferably 3/4 and advantageously 9/10 comprises polymers formed from the compounds of Claim 1, the polymers being advantageously chosen from those referred to in Claims 4 to 13.